# EUROPÄISCHE PATENTSCHRIFT

(11) **EP 3 687 419 B1**
(45) Veröffentlichungstag und Bekanntmachung des Hinweises auf die Patenterteilung: **20.10.2021**
(21) Anmeldenummer: 18756208.7
(22) Anmeldetag: 15.08.2018
(51) Int. Cl.: A61B 17/04, A61B 17/00

(54) **ANKER FÜR CHIRURGISCHE GEWEBEREPARATUREN**
ANCHOR FOR SURGICAL TISSUE REPAIRS
ANCRE POUR RÉPARATIONS TISSULAIRES CHIRURGICALES

(30) Priorität: 27.09.2017 DE 202017105870 U
(43) Veröffentlichungstag der Anmeldung: 05.08.2020
(73) Patentinhaber: H&B Electronic GmbH & Co. KG, 75392 Deckenpfronn (DE)
(72) Erfinder: MORLOK, Tobias, 71159 Mötzingen (DE); WEBER, Wilfried, 72296 Schopfloch (DE); PETRY, Dariusz, 71134 Aidlingen (DE); STAUSS, Wolfgang, 72414 Rangendingen (DE)
(74) Vertreter: Wacker, Jost Oliver
(86) Internationale Anmeldenummer: PCT/EP2018/072146
(87) Internationale Veröffentlichungsnummer: WO 2019/063189

(56) Entgegenhaltungen:
- WO-A2-2004/103162
- CN-Y- 2 392 639
- DE-U1-202015 002 244
- US-A1- 2012 157 761
- US-A1- 2016 007 994

## Beschreibung

Die Erfindung betrifft einen Anker zur chirurgischen Gewebereparatur nach dem Oberbegriff des Anspruchs 1, wie insbesondere zur Reparatur eines Meniskusrisses, eines Bandes oder einer Sehne. Der Anker kann dabei für die Platzierung an einem zu reparierenden Gewebe entlang einer Hohlnadel verschoben werden und ist zudem mit einem Nahtelement verbunden beziehungsweise verbindbar. Ferner weist der Anker wenigstens zwei relativ zueinander verlagerbare Ankerabschnitte auf, die über eine Achsanordnung miteinander verbunden sind. Über diese Achsanordnung können die Ankerabschnitte zwischen einer Einklappstellung und einer Aufklappstellung relativ zueinander verschwenkt werden.

Aus der DE 20 2015 002244 U1 ist eine Ankeranordnung zur chirurgischen Gewebereparatur bekannt. Die Ankeranordnung weist dabei wenigstens einen ersten Anker und einen zweiten Anker auf, die für die jeweilige Platzierung an dem zu reparierenden Gewebe entlang einer Hohlnadel verschoben und aus dieser heraus ausgeworfen werden können. Die wenigstens zwei Anker sind dabei über ein Nahtelement miteinander verbunden. Zur Reparatur des betreffenden Gewebes kann dabei zumindest ein die beiden Anker verbindender Teil des Nahtelementes verkürzt werden, wodurch beispielsweise ein Riss des Gewebes geschlossen werden kann. Dabei sind an den Ankern zwei Ankerabschnitte vorgesehen, die über eine Gelenkachse verbunden sind. Hierdurch können die Anker zumindest teilweise querstehend zur Hohlnadel beziehungsweise zu einer mittels der Hohlnadel erzeugten Durchtrittsöffnung im Gewebe verlagert werden. Durch die Verlagerung der Ankerabschnitte kann somit verhindert werden, dass die Anker bei Zugaufbringung an dem Nahtelement wieder durch die Durchtrittsöffnung zurückgezogen werden.

WO2004/103162A2 beschreibt einen Anker zur Reparatur von Gewebe, der zwei verschwenkbare flügelförmige Ankerelemente aufweist. Jedes der beiden Ankerelemente ist dabei über ein Drehgelenk an einem ersten Kupplungsteil gelagert und weist zudem eine Kulisse auf, entlang der jeweils ein Nocken eines zweiten Kupplungsteils verlagerbar ist. Durch gegenseitige Verlagerung beider Kupplungsteile wird dadurch eine zwangsweise Schwenkbewegung beider Ankerelemente um deren Drehgelenke herum generiert.

Bei den bekannten Ankern werden die Ankerabschnitte dadurch sicher in der Aufklappstellung gehalten, dass sie unter Zug an dem betreffenden Gewebe anliegen.

Die Aufgabe der Erfindung ist es, bei einem gattungsgemäßen Anker die Aufklappstellung der beiden Ankerabschnitte weiter zu stabilisieren.

Diese Aufgabe wird durch einen Anker mit den Merkmalen des Anspruchs 1 gelöst. Dabei weist die Achsanordnung ein seitens des ersten Ankerabschnittes vorgesehenes Achselement und eine seitens des zweiten Ankerabschnittes vorgesehene Führungsausnehmung auf, über die der erste Ankerabschnitt verdrehbar und linear verlagerbar am zweiten Ankerabschnitt gehalten ist. Dabei sind die Ankerabschnitte aus der Einklappstellung mittels einer Drehbewegung in die Aufklappstellung und von dieser mittels einer Linearbewegung in eine drehblockierte Endstellung verlagerbar. Die Verbindung zwischen den beiden Ankerabschnitten weist somit zwei Freiheitsgrade auf, die einerseits eine Drehbewegung zum Öffnen der beiden Ankerabschnitte in die Aufklappstellung und zusätzlich eine Längsbewegung zur gegenseitigen Verlagerung in die Endstellung ermöglicht, in der eine Drehbewegung zurück in die Einklappstellung blockiert ist. Hierdurch kann der Anker nach dem Setzen unabhängig von seiner Positionierung am Gewebe sicher in der geöffneten Endstellung gehalten werden, um einen versehentlichen Wiedereintritt in eine Einstichöffnung des Gewebes ausschließen und über das Nahtelement eine relativ hohe Zugkraft an dem zu reparierenden Gewebeabschnitt aufbringen zu können.

In einer besonders vorteilhaften Ausführungsform ist das Achselement einteilig mit dem ersten Ankerabschnitt ausgeführt. Hierdurch kann die drehbare und in Längsrichtung verlagerbare Verbindung zwischen den beiden Ankerabschnitten auch bei geringen Gesamtabmessungen des Ankers relativ stabil ausgeführt werden.

Alternativ hierzu ist es günstig, wenn das Achselement durch einen mit dem ersten Ankerabschnitt verbindbaren Stift gebildet ist, der insbesondere separat zum ersten Ankerabschnitt hergestellt ist und der in eine in den ersten Ankerabschnitt eingelassene Achsaufnahme eingesteckt werden kann. Hierdurch ist eine relativ kostengünstige Herstellung des Achselementes und des Ankers insgesamt möglich.

Dabei ist es günstig, wenn die Führungsausnehmung eine elastisch aufweitbare Einführöffnung aufweist, über die das Achselement, eingeführt und in der Art einer Rastverbindung am ersten Ankerabschnitt festgelegt werden kann. Die elastisch aufweitbare Einführöffnung ermöglicht dadurch eine einfache Montage des Achselementes am ersten Ankerabschnitt.

Vorteilhafterweise ist die Führungsausnehmung durch zwei parallel angeordnete Längsausnehmungen gebildet, die in zwei zueinander beabstandete Abschnitte des zweiten Ankerabschnittes eingelassen sind. Diese Ankerabschnitte sind dabei so weit voneinander beabstandet, dass das Achselement zwischen diese eingeschoben und durch Drehung um 90° an beiden Enden mit den Längsausnehmungen in Eingriff gebracht werden kann, wobei das Achselement entlang der Längsausnehmungen geführt verlagerbar ist. Diese Ausführungsform ermöglicht eine besonders einfache drehbare und linear verlagerbare Verbindung der beiden Ankerabschnitte.

Zudem ist es günstig, wenn das Achselement und die Führungsausnehmung an beziehungsweise benachbart zu einem Ende des jeweiligen Ankerabschnitts vorgesehen sind, so dass der Anker nach dem Aufklappen beziehungsweise in der Endstellung eine maximierte Längenerstreckung aufweist.

Vorteilhafterweise weist die Führungsausnehmung ein Langloch auf, entlang dessen das Achselement in die Endstellung verlagerbar ist, wodurch nach Erreichen der Aufklappstellung eine sichere gegenseitige Führung der Ankerabschnitte in die Endstellung gewährleistet werden kann.

Ferner ist es günstig, wenn das Achselement in der Einklappstellung am Langloch verrastbar ist, um während der Aufklappbewegung eine reine Dreh- beziehungsweise Schwenkbewegung zwischen den Ankerabschnitten gewährleisten zu können.

Zudem ist es günstig, wenn das Achselement in der Endstellung am Langloch verrastbar ist, um eine versehentliche Verlagerung aus der Endstellung beziehungsweise ein ungewolltes Zusammenklappen der beiden Ankerabschnitte ausschließen zu können.

In einer weiteren vorteilhaften Ausführungsform weist die Führungsausnehmung ein Formschlussmittel auf, das in der Endstellung mit einem Formschlussgegenmittel des Achselementes zusammen wirkt, so dass nach dem Erreichen der Endstellung eine versehentliche Drehbewegung ausgeschlossen werden kann.

Ferner ist es günstig, wenn der erste Ankerabschnitt wenigstens eine erste Anschlagsfläche aufweist, die in der Endstellung mit einer zweiten Anschlagsfläche des zweiten Ankerabschnittes in Anlage kommt. Insbesondere bei Vorhandensein einer gewissen gegeneinander gerichteten Vorspannung können die beiden Ankerabschnitte in der Endstellung auf diese Weise besonders stabil in einer vorgegebenen Position aneinander positioniert werden.

Vorteilhafterweise weisen beide Ankerabschnitte jeweils eine Aufnahmeöffnung auf, an der das Nahtelement jeweils umlenkbar oder festlegbar ist. Durch Anlegen des Ankers an einem Widerlager beziehungsweise an dem zu behandelnden Gewebe und gleichzeitiger Aufbringung von Zugkräften über die Aufnahmeöffnungen können hierdurch Momente und Längsverlagerungskräfte in vorgegebener Wirkrichtung erzeugt werden, durch die der Anker nach dem Setzen sicher aufgeklappt und in die Endstellung verlagert werden kann. Zudem können die Ankerabschnitte über die jeweilige Aufnahmeöffnung unverlierbar am Nahtelement gehalten werden. Auf diese Weise kann sichergestellt werden, dass die Ankerabschnitte insbesondere auch bei einer Beschädigung des Achselementes oder der Führungsausnehmung am Nahtelement verbleiben.

Zudem ist es günstig, wenn die Ankerabschnitte in der Einklappstellung einen zusammen gesetzten Querschnitt aufweisen, der sich vollständig innerhalb eines virtuellen Hüllkreises mit einem Durchmesser von weniger als 2 mm erstreckt, um ein problemloses Einstechen und Durchdringen des zu reparierenden Gewebes mittels einer Nadel zu ermöglichen.

Außerdem ist es günstig, wenn die Ankerabschnitte in der Endstellung eine Gesamtlänge von weniger als 10 mm aufweisen, um einen möglichst störungsfreien und wenig gewebeschädigenden Verbleib des Ankers im Körper des betreffenden Patienten zu gewährleisten.

Ferner wird die oben genannten Aufgabe durch eine Ankersetzanordnung mit einem in einer Nadel aufgenommenen Anker in einer der oben genannten Ausführungsformen und einem Nahtelement gelöst, wobei die wenigstens zwei Ankerabschnitte über das Nahtelement mit einer Zugkraft beaufschlagt werden können, um die Ankerabschnitte beim beziehungsweise nach dem Setzen zunächst mit einem Aufklappmoment, das diese in die Aufklappstellung bewegt, und anschließend mit Längskräften zu beaufschlagen, die die Ankerabschnitte mittels einer Linearbewegung in die Endstellung verbringen.

Dabei ist es günstig, wenn die Zugkraft mittels einer mit dem Nahtelement verbundenen Spannhülse erzeugbar ist, die an der Nadel oder an einem mit der Nadel verbundenen Setzgerät verschiebbar gehalten und mit einer Vorspannung beaufschlagbar ist. Hierdurch können die Ankerabschnitte bereits vor und während des Setzvorganges in die Aufklappstellung vorgespannt werden, so dass sie beim Austritt aus der Nadel unmittelbar aufgeklappt und in der Endstellung aneinander festgelegt werden können. Hierdurch kann ein versehentlicher Wiedereintritt des Ankers in eine beim Setzvorgang hergestellte Eintrittsöffnung des Gewebes im Wesentlichen ausgeschlossen werden.

Vorteilhafterweise können beide Ankerabschnitte neben der durch das Nahtelement aufbringbaren Zugkraft zusätzlich mit einer der Zugkraft versetzt entgegen gerichteten Druckkraft beaufschlagt werden, um ein Aufklappmoment zu erzeugen, durch das die Ankerabschnitte gezielt in die Aufklappstellung verbracht werden können.

Dabei ist es günstig, wenn die Druckkraft mittels eines in der Nadel verlagerbaren Auswerfers erzeugt wird, wodurch die Auswurfbewegung des Auswerfers gleichzeitig zur Erzeugung des Aufklappmomentes genutzt werden kann.

Vorteilhafterweise greift das Nahtelement an den beiden Ankerabschnitten mit schräg aufeinander zu gerichteten Zugkräften an. Durch diese schräge Ausrichtung beispielsweise zweier gegenüber den Ankerabschnitten schräg aufeinander zu laufender Abschnitte des Nahtelementes ist es möglich, neben dem Aufklappmoment auch aufeinander zu gerichtete Kraftkomponenten zu erzeugen, durch die die Ankerabschnitte aus der Aufklappstellung in Längsrichtung in die gegenseitige Endstellung verlagert werden können.

In einer weiteren vorteilhaften Ausführungsform ist ein Aufzugnahtelement zur Verlagerung der Ankerabschnitte aus der Einklappstellung in die Aufklappstellung und/oder die Endstellung sowie ein Haltenahtelement zur Befestigung des Ankers gegenüber dem zu reparierenden Gewebe beziehungsweise zur Verbindung mit einem weiteren Anker vorgesehen. Durch diese Verwendung zweier getrennter Nahtelemente für unterschiedliche Funktionen können diese an den Ankern durchgeschleift und an die jeweilige Funktion angepasst werden. So ist es beispielsweise möglich, das Aufzugnahtelement, das keine Haltefunktion übernehmen muss, durch einen relativ dünnen Faden zu bilden, der insbesondere schnell resorbiert werden kann.

Es wird darauf hingewiesen, dass alle oben beschriebenen Merkmale des erfindungsgemäßen Gegenstandes untereinander austauschbar beziehungsweise kombinierbar sind, sofern ein Austausch oder eine Kombination derselben aus technischen Gründen nicht ausgeschlossen ist.

In den Figuren ist eine beispielhafte Ausführungsform der Erfindung dargestellt. Es zeigen:
- Figur 1: eine perspektivische Ansicht eines in einer teilweise freigeschnittenen Hohlnadel aufgenommenen erfindungsgemäßen Ankers in einer Einklappstellung,
- Figur 2: eine Seitenansicht des Ankers gemäß Figur 1,
- Figur 3: eine Ansicht des Ankers gemäß Figur 1 bei Austritt aus der Hohlnadel,
- Figur 4: eine Ansicht des Ankers gemäß Figur 1 in einer Aufklappstellung,
- Figur 5: eine Ansicht des Ankers gemäß Figur 1 in einer Endstellung,
- Figur 6: eine perspektivische Ansicht des Ankers gemäß Figur 1 mit getrennten Ankerabschnitten,
- Figur 7: eine perspektivische Ansicht des Ankers gemäß Figur 6 beim Einschieben eines Achselementes zwischen zwei Längsausnehmungen,
- Figur 8: eine perspektivische Ansicht des Ankers gemäß Figur 7 bei Eingriff des Achselementes in den Längsausnehmungen,
- Figur 9: eine explodierte perspektivische Ansicht einer alternativen Ausführungsform des Ankers mit einem stiftförmigen Achselement,
- Figur 10: eine perspektivische Ansicht des Ankers gemäß Figur 9 mit zusammengefügten Ankerabschnitten,
- Figur 11: eine perspektivische Ansicht des Ankers gemäß Figur 9 mit montierter Achsanordnung,
- Figur 12: eine perspektivische Ansicht einer weiteren alternativen Ausführungsform des Ankers mit einer verrastbaren Achsanordnung in einem getrennten Zustand,
- Figur 13: eine perspektivische Ansicht des Ankers gemäß Figur 12 in einem zusammengefügten Zustand,
- Figur 14: eine Ansicht einer weiteren alternativen Ausführungsform des Ankers mit einer Formschlussmittel aufweisenden Führungsausnehmung in der Einklappstellung,
- Figur 15: eine Ansicht des Ankers gemäß Figur 14 in der Aufklappstellung,
- Figur 16: eine geschnittene Ansicht des Ankers gemäß Figur 15 beim Anliegen an einem zu reparierenden Gewebe,
- Figur 17: eine geschnittene Ansicht des Ankers gemäß Figur 16 in der Endstellung,
- Figur 18: eine geschnittene Ansicht des Ankers gemäß Figur 15 in der Einklappstellung mit einer alternativen Ausführungsform des Naht-Elementes
- Figur 19: eine geschnittene Ansicht des Ankers gemäß Figur 18 in der Aufklappstellung und
- Figur 20: eine geschnittene Ansicht des Ankers gemäß Figur 18 in der Endstellung.

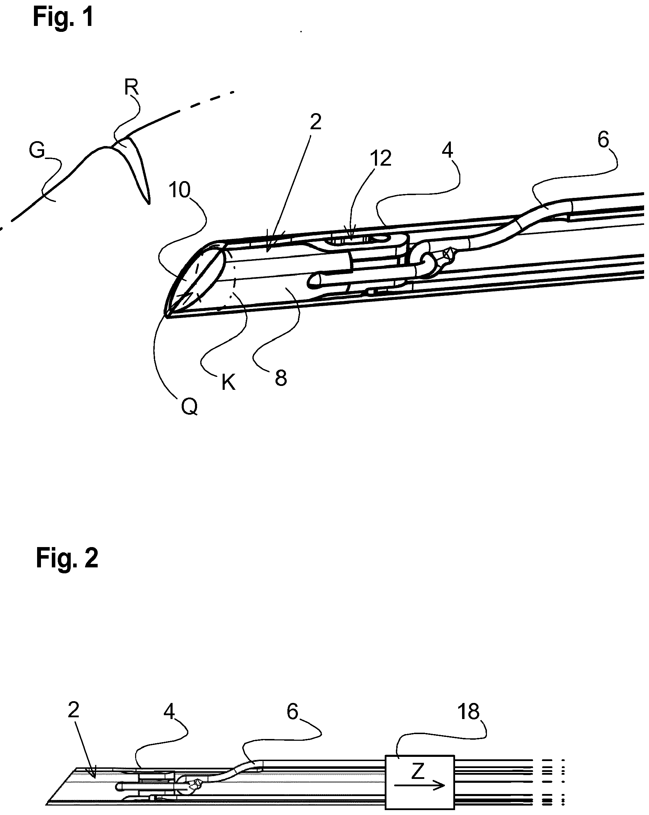

Fig. 1 zeigt einen Anker 2 zur chirurgischen Gewebereparatur eines Meniskusrisses, eines Bandes oder einer Sehne. Hierzu ist der Anker 2 derart ausgebildet, dass er entlang einer Hohlnadel 4 verschoben und aus dieser heraus an dem zu reparierenden Gewebe G positioniert werden kann. Zudem ist der Anker 2 mit einem Nahtelement 6, insbesondere in Form eines Fadens, verbunden, mittels dem der Anker 2 mit einem weiteren Anker verbunden beziehungsweise mit einer Zugkraft beaufschlagt werden kann. Zur Reparatur des betreffenden Gewebes G kann dabei beispielsweise ein zwischen zwei gesetzten Ankern 2 angeordneter Abschnitt des Nahtelementes 6 durch Zusammenziehen verkürzt werden, wodurch beispielsweise ein Riss R des Gewebes G geschlossen werden kann, um ein Zusammenwachsen des Gewebes G an dieser Stelle zu ermöglichen.

Wie aus Figur 1 zu entnehmen ist, weist der Anker 2 einen ersten Ankerabschnitt 8 und einen zweiten Ankerabschnitt 10 auf, die über eine Achsanordnung 12 miteinander verbunden sind. In der dargestellten Einklappstellung liegen die beiden Ankerabschnitte 8, 10 parallel zueinander ausgerichtet aneinander an, sodass sie einen zusammen gesetzten Querschnitt Q bilden, der sich vollständig innerhalb eines virtuellen Hüllkreises K erstreckt, der einen Durchmesser von weniger als 2 mm aufweist. Hierdurch kann der Anker 2 in der Einklappstellung innerhalb der relativ dünnen Hohlnadel 4 aufgenommen und entlang derselben verschoben werden. Vorzugsweise weisen dabei sowohl der zusammengesetzte Querschnitt Q des Ankers 2 als auch die Hohlnadel 4 ein von einer Kreisform abweichendes Profil auf, um den Anker 2 in einer vorbestimmten Drehposition aufnehmen und setzen zu können.

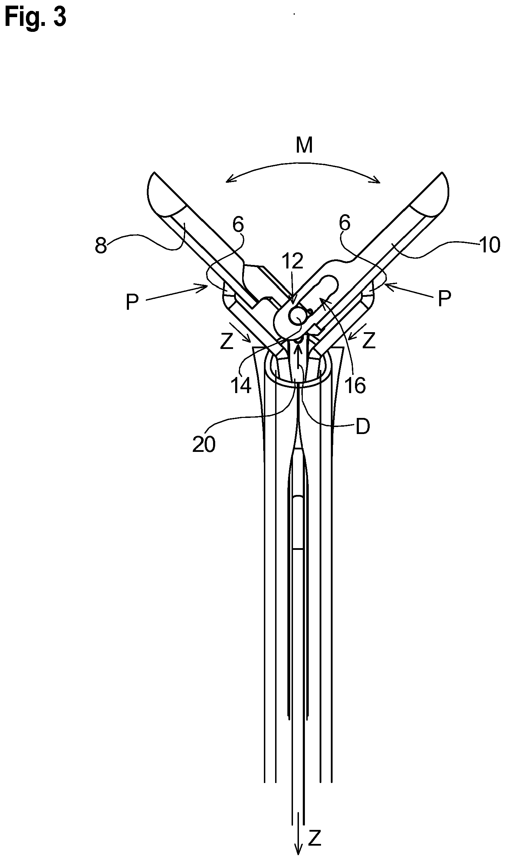

Wie aus Figur 2 zu entnehmen ist, ist an der Hohlnadel 4 eine Spannhülse 18 vorgesehen, über die eine Zugkraft Z auf das Nahtelement 6 aufgebracht werden kann. Alternativ zu der dargestellten Ausführungsform kann die Spannhülse 18 beziehungsweise ein sonstiges mit dem Nahtelement 6 verbundenes und vorspannbares Spannmittel auch an einem mit der Hohlnadel 4 verbundenen Setzgerät vorgesehen sein (nicht dargestellt). Das Nahtelement 6 greift dabei in jedem Fall an beiden Ankerabschnitten 8, 10 an, so dass diese in Richtung einer Aufklappstellung vorgespannt sind. Wie aus Figur 3 zu entnehmen ist, ist das Nahtelement 6 hierzu jeweils an einem Zugpunkt P der Ankerabschnitte 8, 10 befestigt oder durchgeschleift.

Wie aus Figur 3 ferner zu entnehmen ist, weist die Achsanordnung 12 ein Achselement 14 auf, das am ersten Ankerabschnitt 8 vorgesehen ist und das in eine Führungsausnehmung 16 greift, die in den zweiten Ankerabschnitt 10 eingelassen ist. Die Führungsausnehmung 16 ist dabei als Langloch ausgebildet, so dass der erste Ankerabschnitt 8 gegenüber dem zweiten Ankerabschnitt 10 sowohl verdreht als auch linear verlagert werden kann.

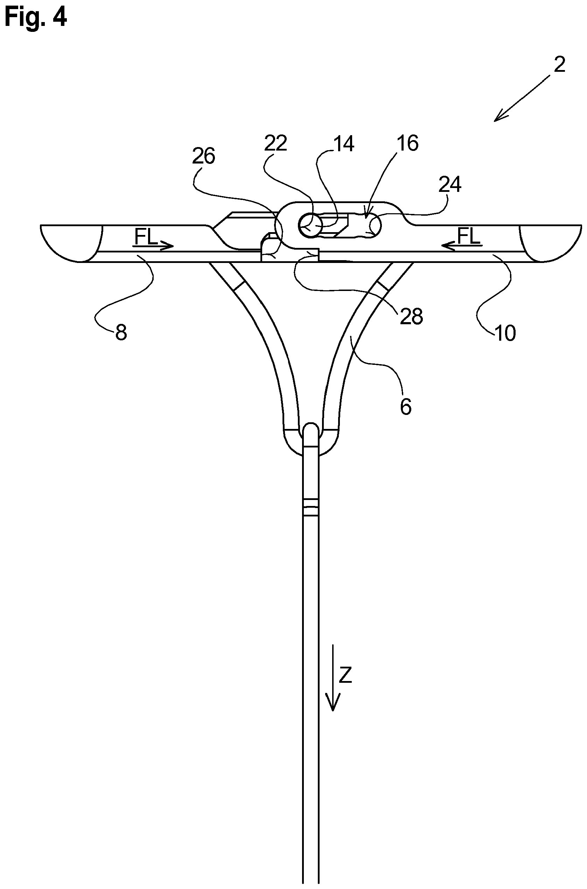

Zum Setzen des Ankers 2 wird dieser über einen Auswerfer 20 mit einer Druckkraft D beaufschlagt und dadurch aus der Hohlnadel 4 heraus geschoben, während gleichzeitig über die Zugpunkte P die proximal gerichtete Zugkraft Z wirkt. Hierdurch entsteht ein Aufklappmoment M, mittels dem die Ankerabschnitte 8, 10 des Ankers 2 beim Austritt aus der Hohlnadel 4 in eine Aufklappstellung gemäß Figur 4 verbracht werden.

Während der Verlagerung der Ankerabschnitte 8, 10 von der Einklappstellung gemäß Figur 1 und 2 in die Aufklappstellung gemäß Figur 4 ist das Achselement 14 dabei in einer an der Führungsausnehmung 16 vorgesehenen ersten Rastaufnahme 22 verrastet, wodurch eine reine Drehbewegung zwischen den Ankerabschnitten 8, 10 gewährleistet ist.

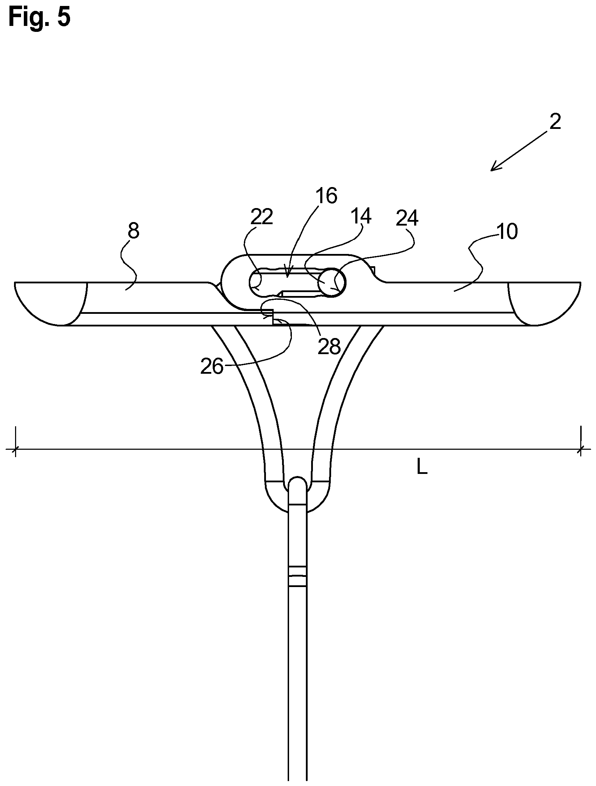

Wie aus Figur 4 ferner zu entnehmen ist, greift das Nahtelement 6 in der Aufklappstellung mit der Zugkraft Z an den beiden Zugpunkten P über zwei Abschnitte an, die sich in proximaler Richtung schräg aufeinander zu erstrecken. Dadurch werden über diese beiden Abschnitte Kraftkomponenten FL an den beiden Ankerabschnitten 8, 10 erzeugt, die in Längsrichtung des Ankers 2 aufeinander zu gerichtet sind. Bei Erreichen eines bestimmten Schwellenwertes dieser längs gerichteten Kraftkomponenten FL löst sich das Achselement 14 aus der Rastverbindung mit der ersten Rastaufnahme 22 und verlagert sich entlang der Führungsausnehmung 16 bis es mit einer von der ersten Rastaufnahme 22 abgewandten zweiten Rastaufnahme 24 der Führungsausnehmung 16 verrastet, wie in Figur 5 dargestellt.

Hierbei werden die beiden Ankerabschnitte 8, 10 durch eine Linearbewegung aufeinander zu verlagert, bis eine stirnseitige erste Anschlagsfläche 26 des ersten Ankerabschnittes 8 mit einer stirnseitigen zweiten Anschlagsfläche 28 des zweiten Ankerabschnittes 10 in Anlage kommt, wie in Figur 5 dargestellt. In dieser Position nehmen die Ankerabschnitte 8, 10 eine Endstellung ein, in der diese durch die beiden aneinander anliegenden Anschlagsflächen 26, 28 sowie durch das in der zweiten Rastaufnahme 24 verrastete Achselement 14 hinsichtlich einer gegenseitigen Drehbewegung blockiert sind.

Nachfolgend sind beispielhaft verschiedene Ausführungsformen des Ankers 2 beziehungsweise der Achsanordnung 12 dargestellt, mittels denen die oben beschriebene Funktionsweise umsetzbar ist.

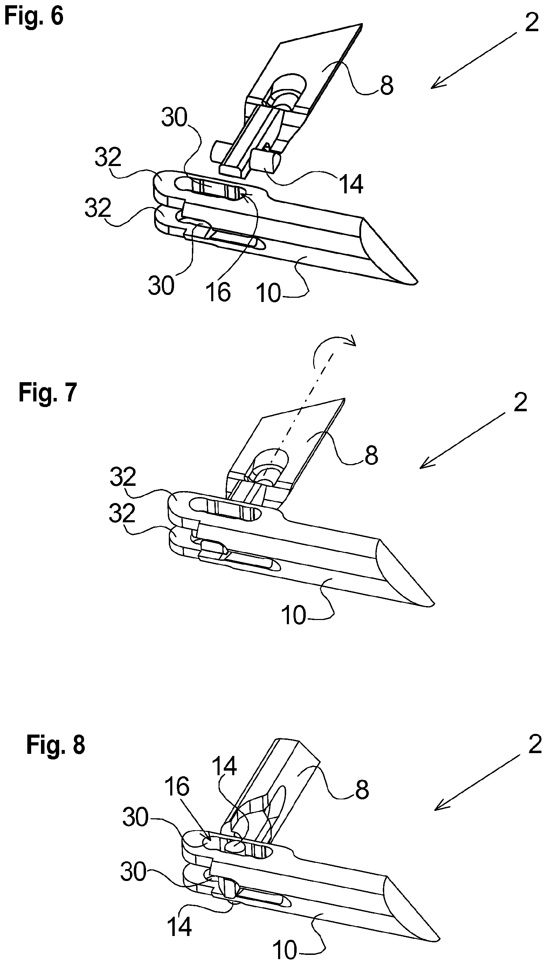

Wie aus Figur 6 zu entnehmen ist, kann das Achselement 14 der Achsanordnung 12 einteilig mit dem ersten Ankerabschnitt 8 ausgeführt sein. Passend hierzu kann die Führungsausnehmung 16 am zweiten Ankerabschnitt 10 durch zwei Längsausnehmungen 30 gebildet sein, die beabstandet parallel zueinander in jeweils einen Teilabschnitt 32 des zweiten Ankerabschnittes 10 eingelassen sind. Diese beiden Teilabschnitte 32 sind dabei derart voneinander beabstandet, dass das Achselement 14 des ersten Ankerabschnittes 8 zwischen beide eingeschoben werden kann, wie in Figur 7 dargestellt. Durch eine anschließende Drehbewegung des ersten Ankerabschnittes 8 über 90° um die eigene Längsachse kann das Achselement 14 an beiden Enden mit den beiden Längsausnehmungen 30 der Führungsausnehmung 16 in Eingriff gebracht werden, wie in Figur 8 dargestellt.

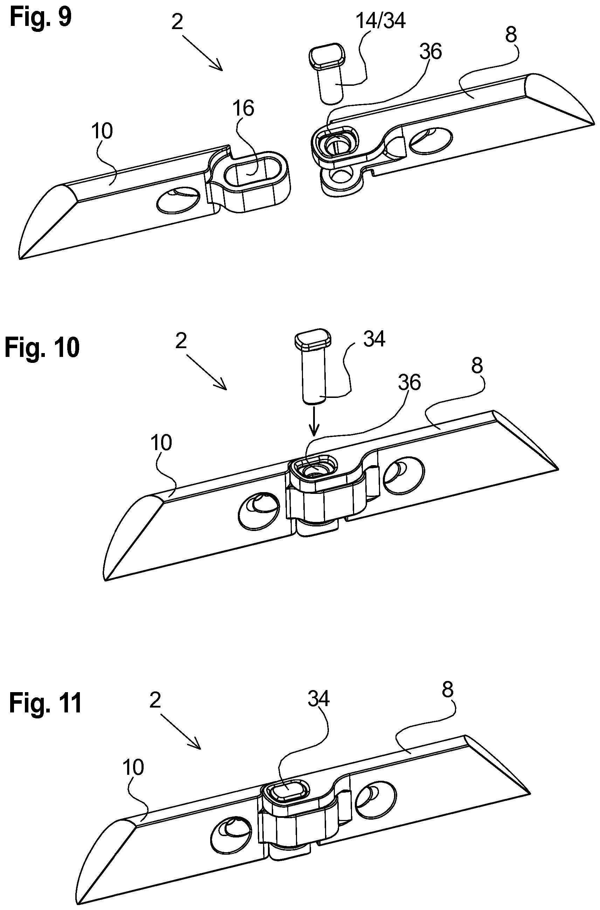

Die Figuren 9 bis 11 zeigen eine alternative Ausführungsform der Achsanordnung 12, bei der das Achselement 14 im Wesentlichen durch einen Stift 34 gebildet ist, der getrennt zu einer Stiftaufnahme 36 des ersten Ankerabschnittes 8 ausgebildet ist. Zur Verbindung beider Ankerabschnitte 8, 10 wird zunächst die Stiftaufnahme 36 benachbart zur Führungsausnehmung 16 des zweiten Ankerabschnittes 10 positioniert. Hierbei kann die Stiftaufnahme 36, wie in Figur 10 beispielhaft dargestellt, durch zwei Aufnahmeöffnungen gebildet sein, zwischen denen die Führungsausnehmung 16 eingeschoben wird. Anschließend wird der Stift 34 in die Stiftaufnahme 36 gesteckt, wobei er gleichzeitig die Führungsausnehmung 16 durchgreift und gemäß Figur 11 eine Gelenkverbindung zwischen beiden Ankerabschnitten 8, 10 bildet, über die die oben beschriebene Dreh- und Linearbewegung zwischen beiden Teilen vorgenommen werden kann.

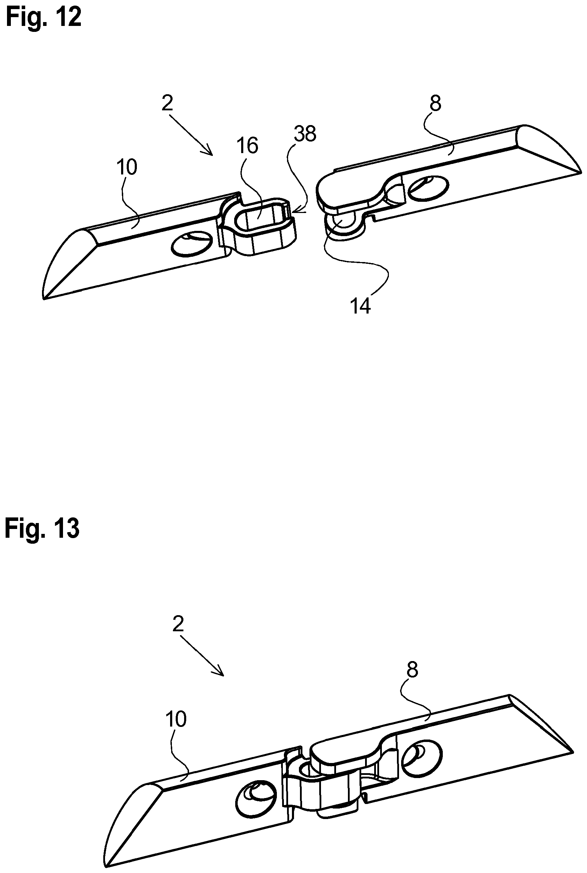

Eine weitere Ausführungsform des Ankers 2 ist aus den Figuren 12 und 13 zu entnehmen. Hierin weist die Führungsausnehmung 16 des zweiten Ankerabschnittes 10 eine elastisch aufweitbare Einführöffnung 38 auf. Über diese Einführöffnung 38 kann das Achselement 14, das entweder wie dargestellt einteilig mit dem ersten Ankerabschnitt 8 verbunden ist oder als separater Stift 34 an diesem angebracht werden kann, in die Führungsausnehmung 16 eingeführt und verdrehbar und linear beweglich aufgenommen werden.

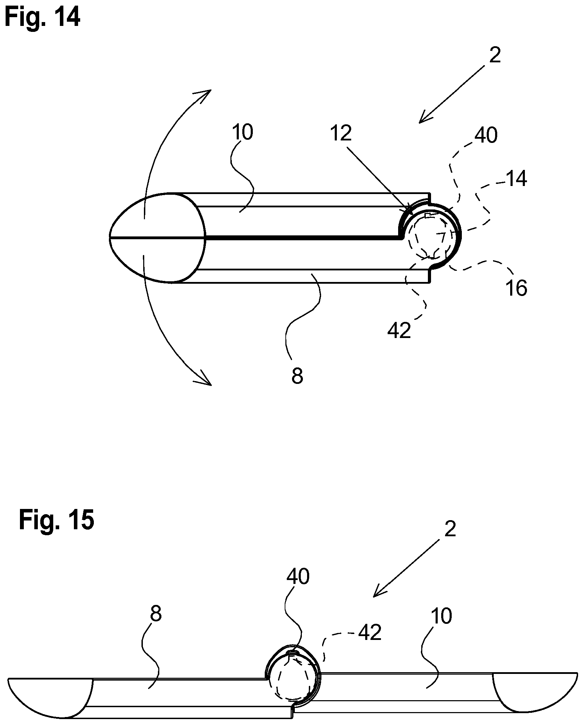

Die Figuren 14 bis 16 zeigen eine weitere Ausführungsform des Ankers 2, bei der die Führungsausnehmung 16 des zweiten Ankerabschnittes 10 im Wesentlichen kreisförmig ausgebildet ist. Zusätzlich weist die Führungsausnehmung 16 dabei ein Formschlussmittel 40 in Form einer randseitigen Ausnehmung auf. Passend hierzu weist das Achselement 14 des ersten Ankerabschnittes 8 einen Querschnitt auf, an dem ein Formschlussgegenmittel 42 in Form eines Nockens absteht.

In der in Figur 14 dargestellten Einklappstellung befindet sich das nockenförmige Formschlussgegenmittel 42 außer Eingriff mit der das Formschlussmittel 40 bildenden Ausnehmung. Hierdurch können die beiden Ankerabschnitte 8, 10 über die durch das Achselement 14 und die Führungsausnehmung 16 gebildete Achsanordnung 12 relativ zueinander in die in Figur 15 dargestellte Aufklappstellung verschwenkt beziehungsweise verdreht werden. In dieser ist das Formschlussmittel 40 des zweiten Ankerabschnittes 10 zunächst benachbart zum Formschlussgegenmittel 42 des ersten Ankerabschnittes 8 angeordnet.

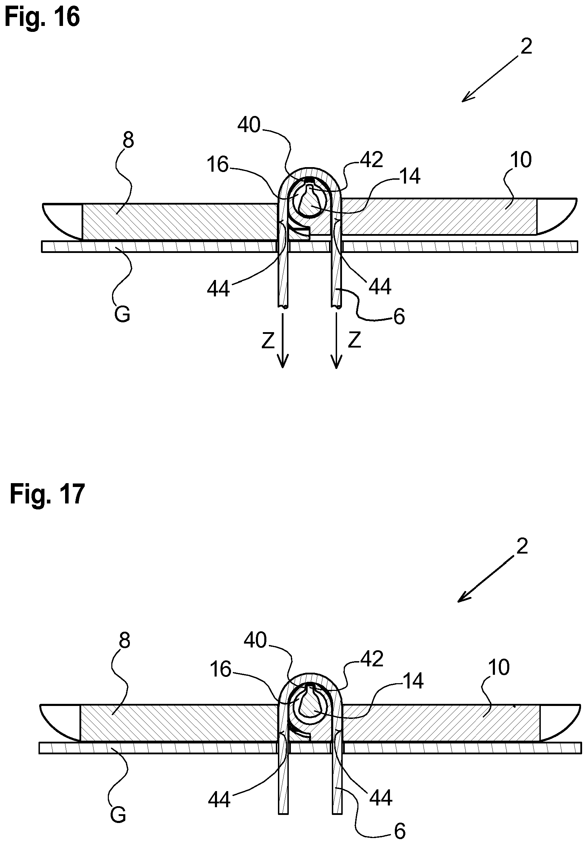

Wie aus Figur 16 zu entnehmen ist, wird der Anker 2 beim Reparaturvorgang durch Aufbringung der Zugkraft Z am Nahtelement 6 in dieser Aufklappstellung am Gewebe G angelegt. Durch weitergehende Aufbringung der Zugkraft Z erfolgt hierbei die Linearbewegung des zweiten Ankerabschnittes 10 gegenüber dem ersten Ankerabschnitt 8 quer zur Längserstreckung beider Ankerabschnitte 8, 10. Hierdurch gelangt das nockenförmige Formschlussgegenmittel 42 des Achselementes 14 in formschlüssigen Eingriff mit dem Formschlussmittel 40 der Führungsausnehmung 16 und damit in die Endstellung des Ankers 2 gemäß Figur 17. In dieser Endstellung ist eine Drehbewegung zwischen den beiden Ankerabschnitten 8, 10 durch die formschlüssige Verbindung zwischen dem Formschlussmittel 40 und dem Formschlussgegenmittel 42 blockiert.

Wie beispielsweise aus Figur 17 ferner zu entnehmen ist, können bei allen oben genannten Ausführungsformen des Ankers 2 zur Anbringung beziehungsweise zum Durchschleifen des Nahtelementes 6 Aufnahmeöffnungen 44 in die Ankerabschnitte 8, 10 eingelassen sein. Die Aufnahmeöffnungen 44 sind dabei derart positioniert, dass das hieran vorgesehene Nahtelement 6 sowohl zur Erzeugung des Aufklappmomentes M als auch zur dauerhaften Befestigung des Ankers 2 am Gewebe G genutzt werden kann.

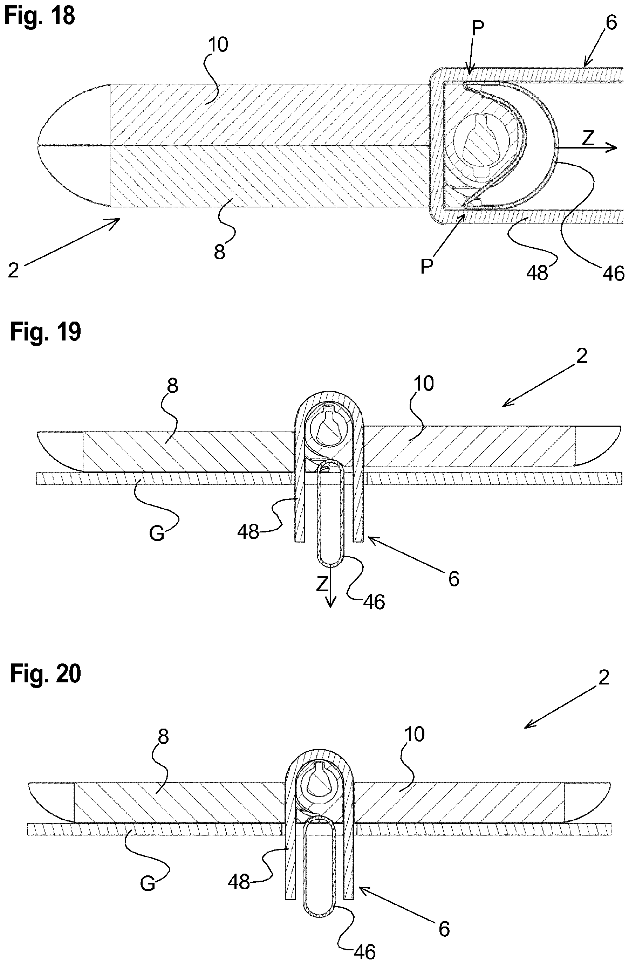

Alternativ hierzu ist es jedoch auch möglich, ein zweiteiliges Nahtelement 6 mit einem Aufzugnahtelement 46 und einem Haltenahtelement 48 vorzusehen, wie in den Figuren 18 bis 20 dargestellt. Hierdurch kann das Aufzugnahtelement 46 an geeigneten Zugpunkten P mit den beiden Ankerabschnitten 8, 10 verbunden beziehungsweise an diesen umgelenkt sein, so dass beim Setzen des Ankers 2 ein ausreichend großes Aufklappmoment erzeugt werden kann, um die Ankerabschnitte sicher in die Aufklappstellung am Gewebe G gemäß Figur 19 zu verbringen. Durch weitere Aufbringung der Zugkraft Z kann über das Aufzugnahtelement 46 und/oder das Haltenahtelement 48 die Linearbewegung in die Endstellung gemäß Figur 20 vorgenommen werden.

Hierbei kann das Material des Aufzugnahtelementes 46 derart gewählt werden, dass es nach erfolgter Anbringung des Ankers 2 am Gewebe G relativ schnell resorbiert werden kann. Im Unterschied hierzu kann das Haltenahtelement 48 dagegen derart am Anker 2 angebracht und durch ein Material gebildet sein, dass es über einen längeren Zeitraum hinweg mit der für die Reparatur des Gewebes G benötigten Zugkraft beaufschlagt werden kann und dabei den Anker 2 sicher in der Endstellung hält.

Es wird darauf hingewiesen, dass alle oben beschriebenen Elemente und Merkmale der verschiedenen Ausführungsformen des erfindungsgemäßen Gegenstandes untereinander austauschbar beziehungsweise kombinierbar sind, sofern ein Austausch oder eine Kombination derselben aus technischen Gründen nicht ausgeschlossen ist.

## Patentansprüche

1. Anker (2) zur chirurgischen Reparatur eines Gewebes (G),
der für die Platzierung an dem zu reparierenden Gewebe (G) entlang einer Hohlnadel (4) verschiebbar und mit einem Nahtelement (6) verbindbar ist,
und der wenigstens zwei relativ zueinander verlagerbare Ankerabschnitte (8, 10) aufweist, die über eine Achsanordnung (12) miteinander verbunden sind, über die die Ankerabschnitte (8,10) zwischen einer Einklappstellung und einer Aufklappstellung relativ zueinander verschwenkbar sind,
**dadurch gekennzeichnet, dass** die Achsanordnung (12) ein seitens des ersten Ankerabschnittes (8) vorgesehenes Achselement (14) und eine seitens des zweiten Ankerabschnittes (10) vorgesehene Führungsausnehmung (16) aufweist, über die der erste Ankerabschnitt (8) verdrehbar und linear verlagerbar gegenüber dem zweiten Ankerabschnitt (10) gehalten ist
und die Ankerabschnitte (8, 10) aus der Einklappstellung mittels einer Drehbewegung in die Aufklappstellung und von dieser mittels einer Linearbewegung in eine drehblockierte Endstellung verlagerbar sind.

2. Anker nach Anspruch 1, **dadurch gekennzeichnet, dass** das Achselement (14) einteilig mit dem ersten Ankerabschnitt (8) ausgeführt ist.

3. Anker nach Anspruch 1, **dadurch gekennzeichnet, dass** das Achselement (14) durch einen mit dem ersten Ankerabschnitt (8) verbindbaren Stift gebildet ist.

4. Anker nach einem der Ansprüche 1 bis 3, **dadurch gekennzeichnet, dass** die Führungsausnehmung (16) eine elastisch aufweitbare Einführöffnung (38) zum Einführen des Achselementes (14) aufweist.

5. Anker nach einem der Ansprüche 1 bis 3, **dadurch gekennzeichnet, dass** die Führungsausnehmung (16) durch zwei parallel angeordnete, beabstandete Längsausnehmungen (30) gebildet ist, zwischen denen das Achselement (14) einschiebbar und durch Drehung um 90° an beiden Enden in geführt verlagerbaren Eingriff bringbar ist.

6. Anker nach einem der Ansprüche 1 bis 5, **dadurch gekennzeichnet, dass** das Achselement (14) und die Führungsausnehmung (16) an einem Ende des jeweiligen Ankerabschnitts (8, 10) vorgesehen sind.

7. Anker nach einem der Ansprüche 1 bis 6, **dadurch gekennzeichnet, dass** die Führungsausnehmung (16) ein Langloch aufweist, entlang dessen das Achselement (14) in die Endstellung verlagerbar ist.

8. Anker nach Anspruch 7, **dadurch gekennzeichnet, dass** das Achselement (14) in der Einklappstellung oder in der Endstellung am Langloch verrastbar ist.

9. Anker nach einem der Ansprüche 1 bis 6, **dadurch gekennzeichnet, dass** die Führungsausnehmung (16) ein Formschlussmittel (40) aufweist, das in der Endstellung mit einem Formschlussgegenmittel (42) des Achselementes (14) zusammen wirkt.

10. Anker nach einem der Ansprüche 1 bis 9, **dadurch gekennzeichnet, dass** der erste Ankerabschnitt (8) wenigstens eine erste Anschlagsfläche (26) aufweist, die in der Endstellung an einer zweiten Anschlagsfläche (28) des zweiten Ankerabschnittes (10) anlegbar ist.

11. Anker nach einem der Ansprüche 1 bis 10, **dadurch gekennzeichnet, dass** beide Ankerabschnitte (8, 10) jeweils eine Aufnahmeöffnung (44) aufweisen, an der das Nahtelement (6) jeweils umlenkbar oder festlegbar ist.

12. Anker nach einem der Ansprüche 1 bis 11, **dadurch gekennzeichnet, dass** die Ankerabschnitte (8, 10) in der Einklappstellung einen zusammen gesetzten Querschnitt (Q) aufweisen, der sich vollständig innerhalb eines virtuellen Hüllkreises (K) mit einem Durchmesser von weniger als 2 mm erstreckt und die Ankerabschnitte (8, 10) in der Endstellung eine Gesamtlänge (L) von weniger als 10 mm aufweisen.

13. Ankersetzanordnung mit einer Nadel, einem in der Nadel aufgenommenen Anker nach einem der Ansprüche 1 bis 12 und einem Nahtelement, **dadurch gekennzeichnet, dass** die wenigstens zwei Ankerabschnitte (8, 10) über das Nahtelement (6) mit einer Zugkraft (Z) beaufschlagbar sind.

14. Ankersetzanordnung nach Anspruch 13, **dadurch gekennzeichnet, dass** die Zugkraft (Z) mittels einer verschiebbaren Spannhülse (18) erzeugbar ist.

15. Ankersetzanordnung nach Anspruch 13 oder 14, **dadurch gekennzeichnet, dass** neben der durch das Nahtelement (6) an beiden Ankerabschnitten (8, 10) aufbringbaren Zugkraft (Z) diese zusätzlich mit einer der Zugkraft (Z) versetzt entgegen gerichteten Druckkraft (D) beaufschlagbar sind, um ein Aufklappmoment (M) zu erzeugen, wobei die Druckkraft (D) mittels eines in der Nadel (4) verlagerbaren Auswerfers (20) erzeugbar ist.

16. Ankersetzanordnung nach einem der Ansprüche 13 bis 15, **dadurch gekennzeichnet, dass** das Nahtelement (6) an den beiden Ankerabschnitten (8, 10) mit schräg aufeinander zu gerichteten Zugkräften (Z) angreift.

17. Ankersetzanordnung nach einem der Ansprüche 13 bis 16, **dadurch gekennzeichnet, dass** ein Aufzugnahtelement (46) zur Verlagerung der Ankerabschnitte (8, 10) aus der Einklappstellung in die Aufklappstellung und/oder die Endstellung sowie ein Haltenahtelement (48) zur Befestigung des Ankers (2) gegenüber dem zu reparierenden Gewebe (G) vorgesehen ist.

## Claims

1. Anchor (2) for surgical repair of a tissue (G),
which anchor, for its placement on the tissue (G) to be repaired, is movable along a hollow needle (4) and connectable to a suture element (6),
and which comprises at least two anchor portions (8, 10), which are connected to each other by an axle arrangement (12), via which the anchor portions (8, 10) are pivotable relative to one another between a retracted position and a deployed position,
**characterised in that** the axle arrangement (12) has an axle element (14) provided on the first anchor portion (8) and a guide recess (16) provided on the second anchor portion (10), by which the first anchor portion (8) is held rotatably and linearly displaceable relative to the second anchor portion (10),
and the anchor portions (8, 10) are displaceable from the retracted position to the deployed position by means of a rotation movement, and are displaceable from the deployed position into a rotationally blocked position by means of a linear movement.

2. Anchor according to claim 1, **characterised in that** the axle element (14) is configured as being of one piece with the anchor portion (8).

3. Anchor according to claim 1, **characterised in that** the axle element (14) is formed by a pin which can be connected to the first anchor portion (8).

4. Anchor according to any one of claims 1 to 3, **characterised in that** the guide recess (16) comprises an elastically expandable inlet opening (38) for inletting the axle element (14).

5. Anchor according to any one of claims 1 to 3, **characterised in that** the guide recess (16) is formed by two longitudinal cut-out openings (30) arranged parallel and at a distance from one another, between which the axle element (14) can be moved and which, by rotation through 90°, can be brought into a guided and displaceable engagement at both ends.

6. Anchor according to any one of claims 1 to 5, **characterised in that** the axle element (14) and the guide recess (16) are provided at one end of the respective anchor portion (8, 10).

7. Anchor according to any one of claims 1 to 6, **characterised in that** the guide recess (16) comprises a longitudinal hole, along which the axle element (14) can be displaced into the end position.

8. Anchor according to claim 7, **characterised in that** the axle element (14) can be locked in the retracted position or in the end position at the longitudinal hole.

9. Anchor according to any one of claims 1 to 6, **characterised in that** the guide recess (16) comprises a positive-fit means (40), which in the end position interacts with a positive-fit counter-means (42) of the axle element (14).

10. Anchor according to any one of claims 1 to 9, **characterised in that** the first anchor portion (8) comprises at least one contact surface (26), which, in the end position can be brought into contact with a second contact surface (28) of the second anchor portion (10).

11. Anchor according to any one of claims 1 to 10, **characterised in that** both anchor portions (8, 10) comprise in each case a receiver opening (44), at which the suture element (6) can in each case be deflected or secured.

12. Anchor according to any one of claims 1 to 11, **characterised in that** the anchor portions (8, 10) in the retracted position exhibit a combined cross-section (Q), which extends entirely within a virtual surface circle (K) with a diameter of less than 2 mm, and the anchor portions (8, 10) exhibit in the end position a total length (L) of less than 10 mm.

13. Anchor set arrangement with a needle, an anchor received in the needle in accordance with any one of claims 1 to 12, and a suture element (6), **characterised in that** the at least two anchor portions (8, 10) can be subjected to a tensile force (Z) by means of the suture element.

14. Anchor set arrangement according to claim 13, **characterised in that** the tensile force (Z) can be created by means of a displaceable tensioning sleeve (18).

15. Anchor set arrangement according to claim 13 or 14, **characterised in that,** in addition to the tensile force (Z) which can be applied by the suture element (6) to both anchor portions (8, 10), they can additionally be subjected to a pressure force (D) directed offset towards the tensile force (Z), in order to produce a deployment torque moment force (M), wherein the pressure force (D) can be produced by means of an ejector unit (20) which can be displaced in the needle (4).

16. Anchor set arrangement according to any one of claims 13 to 15, **characterised in that** the suture element (6) engages at the two anchor portions (8, 10) with tensile forces (Z) directed obliquely towards one another,

17. Anchor set arrangement according to any one of claims 13 to 16, **characterised in that** a draw-on suture element (46) is provided for the displacement of the anchor portions (8, 10) out of the retracted position into the deployed position and/or into the end position, as well as a holding suture element (48) for securing the anchor (2) in relation to the tissue (G) which is to be repaired.

## Revendications

1. Ancre (2) pour la réparation chirurgicale d'un tissu (G),
qui peut être déplacée le long d'une aiguille creuse (4) pour être placée sur le tissu (G) à réparer et peut être reliée à un élément de suture (6),
et qui comporte au moins deux sections d'ancre (8, 10) déplaçables relativement l'une par rapport à l'autre, qui sont reliées l'une à l'autre par un agencement d'essieu (12) au moyen duquel les sections d'ancre (8,10) peuvent pivoter relativement l'une par rapport à l'autre entre une position repliée et une position dépliée,
**caractérisée en ce que** l'agencement d'essieu (12) comporte un élément d'essieu (14) prévu du côté de la première section d'ancre (8) et un évidement de guidage (16) prévu du côté de la deuxième section d'ancre (10) au moyen duquel la première section d'ancre (8) est maintenue de manière à pouvoir tourner et être déplacé linéairement par rapport à la deuxième section d'ancre (10)
et les sections d'ancre (8, 10) sont déplaçables de la position repliée dans la position dépliée au moyen d'un mouvement de rotation et de la position dépliée dans une position finale bloquée en rotation au moyen d'un mouvement linéaire.

2. Ancre selon la revendication 1, **caractérisée en ce que** l'élément d'essieu (14) est réalisé d'une pièce avec la première section d'ancre (8).

3. Ancre selon la revendication 1, **caractérisée en ce que** l'élément d'essieu (14) est formé par une tige qui peut être reliée à la première section d'ancre (8).

4. Ancre selon l'une des revendications 1 à 3, **caractérisée en ce que** l'évidement de guidage (16) comporte une ouverture d'insertion (38) extensible élastiquement pour l'insertion de l'élément d'essieu (14).

5. Ancre selon l'une des revendications 1 à 3, **caractérisée en ce que** l'évidement de guidage (16) est formé par deux évidements longitudinaux (30) agencés parallèlement et espacés, entre lesquels l'élément d'essieu (14) peut être inséré et mis en engagement déplaçable guidé par rotation de 90° aux deux extrémités.

6. Ancre selon l'une des revendications 1 à 5, **caractérisée en ce que** l'élément d'essieu (14) et l'évidement de guidage (16) sont prévus à une extrémité de la section d'ancre respective (8, 10).

7. Ancre selon l'une des revendications 1 à 6, **caractérisée en ce que** l'évidement de guidage (16) comporte un trou oblong le long duquel l'élément d'essieu (14) peut être déplacé dans la position finale.

8. Ancre selon la revendication 7, **caractérisée en ce que** l'élément d'essieu (14) peut être encliqueté au trou oblong dans la position repliée ou dans la position finale.

9. Ancre selon l'une des revendications 1 à 6, **caractérisée en ce que** l'évidement de guidage (16) comporte un moyen d'ajustement de forme (40) qui coopère dans la position finale avec un contre-moyen d'ajustement de forme (42) de l'élément d'essieu (14).

10. Ancre selon l'une des revendications 1 à 9, **caractérisée en ce que** la première section d'ancre (8) comporte au moins une première surface de butée (26) qui, dans la position finale, peut être placée contre une deuxième surface de butée (28) de la deuxième section d'ancre (10).

11. Ancre selon l'une des revendications 1 à 10, **caractérisée en ce que** les deux sections d'ancre (8, 10) comportent chacune une ouverture de réception (44) à laquelle l'élément de suture (6) peut respectivement être dévié ou fixé.

12. Ancre selon l'une des revendications 1 à 11, **caractérisée en ce que** les sections d'ancre (8, 10) en position repliée comportent une section transversale composée (Q) s'étendant entièrement à l'intérieur d'un cercle enveloppant virtuel (K) de diamètre de moins de 2 mm et les sections d'ancre (8, 10) ont une longueur totale (L) inférieure à 10 mm en position finale.

13. Dispositif de pose d'ancre avec une aiguille, une ancre reçue dans l'aiguille selon l'une des revendications 1 à 12 et un élément de suture, **caractérisé en ce que** les au moins deux sections d'ancre (8, 10) peuvent être soumises à une force de traction (Z) via l'élément de suture (6).

14. Dispositif de pose d'ancre selon la revendication 13, **caractérisé en ce que** la force de traction (Z) peut être générée au moyen d'une douille de serrage déplaçable (18).

15. Dispositif de pose d'ancre selon la revendication 13 ou 14, **caractérisé en ce qu'**en plus de la force de traction (Z) applicable aux deux sections d'ancre (8, 10) par l'élément de suture (6), une force de compression (D) décalée et opposée à la force de traction (Z) peut également être appliquée aux sections d'ancre pour générer un moment de dépliage (M), la force de compression (D) pouvant être générée au moyen d'un éjecteur (20) déplaçable dans l'aiguille (4).

16. Dispositif de pose d'ancre selon l'une des revendications 13 à 15, **caractérisé en ce que** l'élément de suture (6) engage les deux sections d'ancre (8, 10) avec des forces de traction (Z) dirigées obliquement l'une vers l'autre.

17. Dispositif de pose d'ancre selon l'une des revendications 13 à 16, **caractérisé en ce qu'**un élément d'ouverture de suture (46) est prévu pour déplacer les sections d'ancres (8, 10) de la position repliée à la position dépliée et/ou à la position finale et un élément de maintien de suture (48) est prévu pour fixer l'ancre (2) par rapport au tissu à réparer (G).
